# EUROPEAN PATENT APPLICATION

(11) **EP 2 947 458 A1**
(43) Date of publication of application: **25.11.2015**
(21) Application number: 13871622.0
(22) Date of filing: 26.12.2013
(51) Int. Cl.: G01N 33/53, G01N 33/48, G01N 33/545

(54) **METHOD FOR IMMUNOLOGICALLY ASSAYING HEMOGLOBIN A1C IN SPECIMEN**

(30) Priority: 16.01.2013 JP 2013005130
(71) Applicant: Fujirebio Inc., Tokyo 163-0410 (JP)
(72) Inventor: TETSUMOTO, Toru, Tokyo 103-0007 (JP); HIRATA, Minoru, Tokyo 103-0007 (JP)
(74) Representative: Keirstead, Tanis Evelyne
(86) International application number: PCT/JP2013/084905
(87) International publication number: WO 2014/112318

(57) **Abstract**

Means that enables an immunoassay of hemoglobin A1c in a whole blood sample without pretreatment of the sample is disclosed. The immunoassay of hemoglobin A1c in a sample according to the present invention comprises bringing latex particles into contact with a non-pretreated whole blood sample in a hypotonic solution, and then bringing hemoglobin A1c adsorbed on the latex particles into contact with an anti-hemoglobin A1c antibody. The immunoassay is preferably carried out by an agglutination method. The hypotonic solution is, for example, a buffer containing a Good's buffer at a concentration of 0.02 to 0.2 mol/L, which Good's buffer has the maximum buffer capacity in the neutral to alkaline region.

## Description

### TECHNICAL FIELD

The present invention relates to a method of immunoassay of hemoglobin A1c in a sample.

### BACKGROUND ART

Hemoglobin (Hb) has a heterotetramer structure consisting of two α-chains and two β-chains, and hemoglobin A1c (HbA1c) is a glycosylated hemoglobin generated by nonenzymatic glycosylation at the α-amino group of the N-terminal valine in each β-chain. The blood level of HbA1c reflects the status of blood glucose control in a relatively long time scale in diabetes. Thus, measurement of HbA1c is clinically extremely important for knowing the status of blood glucose control, and measurement of HbA1c has been carried out for, e.g., monitoring of therapeutic effects on diabetes. In the diagnostic criteria for diabetes revised in July 2010, certain association of the HbAlc value with development of retinopathy was noticed, and an HbA1c level of not less than 6.1% (JDS value) was included in the diagnostic criteria (Non-patent Document 1). In April 2012, the value was changed from not less than 6.1% (JDS value) to not less than 6.5% (NGSP value) to follow the world standard.

In recent years, development of immunoassay methods using autoanalyzers for measurement of HbA1c has been promoted due to the limitation of multiple sample processing by the HPLC method mainly in examination centers. In addition, an apparatus that collects blood cells from the blood cell layer of centrifuged blood and prepares a hemolysate therefrom with a sample diluent has been developed, so that automation has proceeded.

On the other hand, it is also a fact that there are facilities avoiding immunoassays because of difficulty in carrying out pretreatments such as centrifugation and hemolysis and dilution in the facilities. Although the automation has proceeded, pretreatments are still required in the immunoassay, unlike the HPLC method. For example, in the case where whole blood is diluted and hemolyzed without centrifugation to measure HbA1c, erythrocytes in a blood collection tube sediment when the tube is left to stand after blood collection. However, in the HbA1c measurement, it is hemoglobin present in erythrocytes that is to be measured. If sampling from the liquid surface is carried out like the case of a serum sample, such sedimented erythrocytes cannot be collected. Thus, the sampling has to be carried out immediately after mixing by inversion, or by inserting a needle into the vicinity of the bottom of the tube. Moreover, since the N-terminus of the β-chain, which is the specific site of HbA1c, is embedded in the higher order structure of the protein molecule, HbA1c in the native state is known to be nonreactive with monoclonal antibodies specific to HbA1c. This fact prevents omission of pretreatments such as addition of a denaturant to HbA1c to alter the higher order structure of the protein or enzymatic cleavage of the N-terminal region of the β-chain to enable HbA1c to react with an antibody.

### PRIOR ART DOCUMENT(S)

### PATENT DOCUMENT(S)

Patent Document 1: JP 2677753 B

### NON-PATENT DOCUMENT(S)

Non-patent Document 1: Journal of the Japan Diabetes Society, 53(6), 450, 2010

### SUMMARY OF THE INVENTION

### PROBLEMS TO BE SOLVED BY THE INVENTION

An object of the present invention is to provide means that enables an immunoassay of HbA1c in a whole blood sample without carrying out pretreatment of the sample.

### MEANS FOR SOLVING THE PROBLEMS

As a result of intensive study, the present inventors discovered that, by adding a non-pretreated whole blood sample as it is to a latex reagent in which latex particles are suspended in a hypoosmotic buffer, hemolysis of the whole blood sample and adsorption of hemoglobin to the surface of the latex particles can be allowed to occur without being adversely affected by the presence of excessive hemoglobin, and HbA1c can be detected using an anti-HbA1c antibody without hemolysis treatment or denaturation treatment, thereby completing the present invention.

That is, the present invention provides a method of immunoassay of hemoglobin A1c in a sample, said method comprising bringing latex particles into contact with a non-pretreated whole blood sample in a hypotonic solution, and then bringing hemoglobin A1c adsorbed on the latex particles into contact with an anti-hemoglobin A1c antibody.

### EFFECT OF THE INVENTION

By the present invention, in the single step of bringing, e.g., a hypotonic solution containing latex particles into contact with a whole blood sample, both hemolysis of the whole blood and adsorption of the whole blood on the latex particles can be achieved. Conventional HbA1c immunoassays require a dilution/hemolysis treatment and a special denaturation treatment, whereas the present invention can omit such pretreatment steps. In particular, some of general-purpose autoanalyzers use a reaction vessel (cell) only for dilution and hemolysis of a sample, but, in the present invention, such a reaction vessel (cell) is not required, so that the sample processing capacity is remarkably high. By the present invention, the processing time can be reduced, and the amount of consumables such as reagents, tubes, and chips, which are conventionally used for pretreatments, can also be reduced, so that the amount of medical wastes and the cost of the measurement can be reduced.

### BRIEF DESCRIPTION OF THE DRAWINGS

Fig. 1 shows the results of measurement of the absorbance of each of a standard HbA1c solution (No. 1), whole blood solution after hemolysis treatment (No. 2), and a plasma solution (No. 3). Taking the performance of the spectrophotometer into account, the concentration of each sample was adjusted to half of the normally used concentration before the measurement.
Fig. 2 shows the calibration curves prepared with two kinds of standard samples.
Fig. 3 shows the changes in the absorbances during the reaction process observed when the absorbances of a diluted hemolyzed blood cell sample and a whole blood sample, which was a whole blood sample without pretreatment, were measured.
Fig. 4 shows the calibration curves prepared with standard samples prepared by adding γ-globulin to a reference standard at different concentrations.

### MODE FOR CARRYING OUT THE INVENTION

The method of the present invention is characterized in that collected whole blood is directly used without pretreatment. The "pretreatment" is a treatment carried out on a sample before bringing the sample into contact with latex particles, and includes hemolysis treatment and protein denaturation treatment. Addition of EDTA and addition of heparin for prevention of hemolysis of collected blood are not included in the "pretreatment" in the present invention.

The hypotonic solution used in the present invention is not limited as long as it is an aqueous liquid whose osmotic pressure is low enough to allow hemolysis of whole blood. Since the osmotic pressure of blood is about 280 mOsm/kg H₂O, a liquid whose osmotic pressure is sufficiently lower than this (for example, not more than 100 mOsm/kg H₂O) can be used as the hypotonic solution. For example, pure water may be used as the hypotonic solution, or a hypoosmotic buffer containing an appropriate concentration (for example, about 0.02 to 0.2 mol/L) of a Good's buffer having the maximum buffer capacity in the neutral to alkaline region may be preferably used. Specific examples of such a Good's buffer include the following, but the Good's buffer is not limited to these.
BES [N,N-Bis(2-hydroxyethyl)-2-aminoethanesulfonic acid]
MOPS [3-Morpholinopropanesulfonic acid]
TES [N-Tris(hydroxymethyl)methyl-2-aminoethanesulfonic acid]
HEPES [4-(2-hydroxyethyl)-1-piperazineethanesulfonic acid]
DIPSO [3-[N,N-Bis(2-hydroxyethyl)amino]-2-hydroxy-1-propanesulfonic acid]
TAPSO [3-(N-tris[Hydroxymethyl]methylamino)-2-hydroxypropanesulfonic acid]
POPSO [Piperazine-1,4-bis(2-hydroxypropanesulfonic acid)]
HEPPSO [N-(Hydroxyethyl)piperazine-N'-2-hydroxypropanesulfonic acid]
EPPS [3-[4-(2-Hydroxyethyl)-1-piperazinyl]propanesulfonic acid]
Tricine [N-[Tris(hydroxymethyl)methyl]glycine]
Bicine [N,N-Bis(2-hydroxyethyl)glycine]
TAPS [N-Tris(hydroxymethyl)methyl-3-aminopropanesulfonic acid]

The latex particles used in the present invention are unsensitized latex particles, that is, latex particles on which proteins such as an antigen are not adsorbed. The latex particles *per se* may be the same as those used in a known HbA1c immunoagglutination assay kit.

In the method of the present invention, a non-pretreated whole blood sample is brought into contact with latex particles in a hypotonic solution to allow adsorption of hemoglobin (which includes normal hemoglobin and HbA1c, which is a glycosylated hemoglobin) on the surface of the latex particles. The whole blood sample contains an excessive amount of hemoglobin relative to the amount of the latex particles. Since there is no substantial difference in adsorption to unsensitized latex between normal hemoglobin and glycosylated hemoglobin HbA1c, the amount of HbA1c adsorbed on the surface of the latex particles directly reflects the ratio of HbA1c to the total amount of Hb present in the whole blood sample. In the case of a reaction system using 100 to 200 µL of a hypotonic solution containing latex particles (particle diameter, 50 to 200 nm; concentration, 0.05 to 0.2%), 1 µL to 2 µL of a whole blood sample may be added. The length of time of bringing the whole blood sample into contact with the latex particles in the hypotonic solution may be about 1 to 5 minutes at room temperature or 37°C in the case of a reaction system in the above-described scale. A conventional immunoassay of HbA1c uses an anti-HbA1c antibody that recognizes the N-terminal region of the β-chain, which is specific to HbA1c. Since the N-terminal region of the β-chain is embedded in the higher order structure of the HbA1c molecule, the immunoassay of HbA1c with the anti-HbA1c antibody usually requires denaturation treatment of the sample. In contrast, in cases where HbA1c is allowed to be adsorbed to latex, the N-terminal region of the β-chain of the HbA1c is exposed, so that an immunoassay with an anti-HbA1c antibody can be carried out without performing a special denaturation treatment.

Subsequently, the HbA1c adsorbed on the latex particles is brought into contact with an anti-HbA1c antibody, and the HbA1c in the sample is measured by an immunoassay. Methods of immunoassays are well known in the art, and any of the methods may be used in the method of the present invention. From the viewpoint of the reaction mode, immunoassays can be classified into the sandwich method, competition method, agglutination method, immunochromatography method, Western blotting method, and the like. From the viewpoint of the label, immunoassays can be classified into radioimmunoassay, fluorescence immunoassay, enzyme immunoassay (EIA), biotin immunoassay, and the like. All of these are included in the "immunoassay" in the present invention, and any of these may be employed in the method of the present invention. Although the most preferred method of the immunoassay in the present invention is the agglutination method, methods other than the agglutination method may also be used since HbA1c can be measured by bringing a hypotonic solution containing latex particles into contact with a whole blood sample that has not been subjected to hemolysis treatment, thereby allowing HbA1c to be adsorbed on the surface of the latex particles, and then carrying out an ordinary immunoassay using the resulting reaction liquid containing the particles as a sample. In the present invention, the term "measurement" includes qualitative detection, quantification, and semi-quantification.

For example, in the agglutination method, HbA1c adsorbed on the surface of latex particles is brought into contact with an anti-HbA1c antibody. The anti-HbA1c antibody then causes binding of the latex particles to each other via HbA1c to cause agglutination of the latex particles, resulting in an increase in the turbidity of the reaction liquid. By visual observation of the turbidity of the reaction liquid, the presence of HbA1c can be detected (qualitative detection). The turbidity may also be optically measured using an autoanalyzer or the like for quantification of the amount of HbA1c. In such a case, standard samples with known HbA1c concentrations may be provided, and each standard sample may be subjected to optical measurement of the turbidity, followed by plotting the relationship between the measured values of turbidity and the concentrations of HbA1c, thereby preparing a calibration curve. By applying the measured value of a whole blood sample to this calibration curve, the amount of HbA1c in the whole blood sample can be determined.

In cases where HbA1c is measured by the sandwich method, an anti-Hb antibody (which binds to total hemoglobins including HbA1c) may be immobilized on a solid phase, and a reaction liquid prepared by bringing a hypotonic solution containing latex particles into contact with a whole blood sample may be reacted with the immobilized antibody, followed by washing the resultant, reacting a labeled anti-HbA1c antibody therewith, washing the resultant, and then measuring the labeled antibody bound to the solid phase based on the signal from the label. The measurement may also be carried out by using an anti-HbA1c antibody as the immobilized antibody and using an anti-Hb antibody as the labeled antibody. Standard samples with known HbA1c concentrations are subjected to the measurement, and the relationship between the signal intensities and the HbA1c concentrations is plotted to prepare a calibration curve. By applying the measured value of a whole blood sample to this calibration curve, the amount of HbA1c in the whole blood sample can be quantified.

In cases where HbA1c is measured by lateral flow immunochromatography, which is an example of the sandwich method, the immunochromatography device may have the following constitution.

Usually, a matrix composed of a porous material such as a nitrocellulose membrane is formed into a belt shape. On this matrix, an anti-HbA1c monoclonal antibody is immobilized to provide a detection zone, and a labeled anti-Hb monoclonal antibody is spotted upstream thereof (upstream in the direction of the flow of the developer described later) to provide a labeled reagent zone. The labeled reagent zone is usually constituted by a porous pad on which a labeled antibody is spotted. At the upstream end of the matrix, a developer tank storing a developer is provided. In cases where an enzyme is used as the label, a substrate zone on which the substrate of the label enzyme is spotted may be provided upstream of the labeled reagent zone.

In the measurement, a reaction liquid obtained by allowing HbA1c to be adsorbed on the surface of the latex particles is added to the labeled reagent zone together with the latex particles contained therein. Thereafter, by breaking the developer tank, the developer is allowed to flow downstream by capillary action of the matrix. The developer sequentially passes through the substrate zone and the labeled reagent zone, and thus the substrate, the labeled antibody, and the latex particles on which hemoglobin is adsorbed flow downstream together with the developer. During this process, the hemoglobin on the surface of the latex particles binds to the labeled anti-Hb antibody due to antigen-antibody reaction. When the immune complex reaches the detection zone, the labeled antibody is captured by the anti-HbA1c antibody in the detection zone through HbA1c, which is contained in the hemoglobins on the surface of the latex particles. Since the substrate is flowing together, the detection zone, in which the labeled antibody is captured, is colored. This coloring may be visually observed to make a determination. Based on the presence/absence of the coloring, qualitative detection of HbA1c is possible. In addition, rough quantification is also possible based on the intensity of the coloring in the detection zone.

In addition to the above, an immunochromatography device in which an anti-Hb antibody is used as the antibody to be immobilized on the detection zone and an anti-HbA1c antibody is used as the labeled antibody to be spotted on the labeled reagent zone may also be used. An antibody against the labeled substance may be spotted next to the detection zone to provide a control zone in order to enable confirmation of appropriate developing of the labeled antibody.

Anti-HbA1c antibodies are known, and various HbA1c immunoassay kits using anti-HbA1c antibodies are commercially available. In the present invention, such known anti-HbA1c antibodies may be used. Since antibodies can be prepared by well-known conventional methods, an antibody which can distinguish HbA1c from non-glycosylated, normal hemoglobin to specifically recognize HbA1c may be prepared and used. As described above, the site specific to HbA1c is the N-terminal region of the β-chain. By immunizing an animal (excluding human) using the N-terminal region of the β-chain of HbA1c as an immunogen to induce antibodies against HbA1c in the body of the animal, polyclonal antibodies against HbA1c can be obtained from serum of the animal. In addition, spleen cells may be recovered from the immunized animal, and the conventional hybridoma method may be carried out to prepare an anti-HbA1c monoclonal antibody that specifically binds to HbA1c. From the viewpoint of reproducibility and the like in the immunoassay, an anti-HbA1c monoclonal antibody is preferably used in the present invention.

Specific examples of the method for preparing the N-terminal region of the β-chain of HbA1c to be used as the immunogen and the method for preparing a monoclonal antibody against the region are described in detail in, for example, Patent Document 1 (JP 2677753 B), and those skilled in the art can appropriately prepare them. The processes are concretely described below.

The N-terminal region of the β-chain of HbA1c to be used as the immunogen can be prepared by synthesizing a peptide corresponding to the N-terminal region of the hemoglobin β-chain and nonenzymatically binding glucose to the α-amino group of the N-terminal amino acid residue, valine. The peptide to be subjected to the glycosylation treatment can be synthesized by a conventional method using a commercially available peptide synthesizer. The N-terminal region of the hemoglobin β-chain to be synthesized may be several residues in size. SEQ ID NO:1 shows the sequence of the 5 residues at the N-terminus of the human hemoglobin β-chain. The N-terminal peptide can be glycosylated by dissolving the prepared N-terminal peptide in acetic acid, adding twice the molar amount of glucose to the resulting solution in the presence of pyridine, and then stirring the resulting mixture at room temperature for about 10 days. Since glycosylation of the peptide reduces the retention time in HPLC, the progress of the glycosylation reaction can be checked by HPLC. The conditions of the analysis by HPLC may be, for example, as follows.
Column: TSKgel, ODS-120A (4.6 x 250 mm, manufactured by Tosoh Corporation) Apparatus: Shimadzu HPLC System
Mobile phase: Linear gradient from 10% acetonitrile/0.1% trifluoroacetic acid to 60% acetonitrile/0.1% trifluoroacetic acid
Flow rate: 0.8 mL/min
Time: 25 min
Monitoring: Absorbance at 280 nm

The obtained glycosylated peptide (N-terminal region of the HbA1c β-chain) can be purified by HPLC under, for example, the following separation conditions.
Column: TSKgel, ODS-120A (21.5 x 300 mm, manufactured by Tosoh Corporation)
Mobile phase: Linear gradient from 10% acetonitrile/0.1% trifluoroacetic acid to 60% acetonitrile/0.1% trifluoroacetic acid
Flow rate: 5 mL/min
Monitoring: Absorbance at 280 nm

From the purified peptide, the cysteine protecting group added during the chemical peptide synthesis process may be eliminated by a conventional method, and HPLC purification may be carried out again under the same separation conditions as described above (except that the absorbance for monitoring may be 215 nm), to provide an immunogen.

Since the N-terminal region of the HbA1c β-chain produced as described above is several residues in size, when the peptide is used as an immunogen, the peptide may be bound to an appropriate carrier protein (e.g., thyroglobulin, edestin or the like) and mixed with an appropriate adjuvant (e.g., complete Freund adjuvant, incomplete Freund adjuvant or the like). The resulting mixture may then be used for immunization of an animal (excluding human). By this, as described above, antibodies against the immunogen can be induced in the body of the animal, and spleen cells can then be recovered from the animal, followed by preparing a monoclonal antibody by the hybridoma method.

Screening of an anti-HbA1c antibody having a desirable specificity to HbA1c can be carried out by, for example, providing a plate on which normal hemoglobin is immobilized and a plate on which HbA1c is immobilized, and investigating reactivity of a plurality of lines of obtained antibodies with both plates. An antibody whose reactivity with the normal hemoglobin is about not more than that of the background and whose reactivity with HbA1c is high can be preferably used as an HbA1c-specific antibody.

Quantification of HbA1c is possible by preparing appropriate standard samples and then preparing a calibration curve therewith. In the present invention, a whole blood sample is used. The amount of plasma contained in a whole blood sample is larger than that in a sample subjected to blood-cell hemolysis treatment. In the case where such a sample is used, the calibration curve may be prepared using HbA1c standard samples prepared by adding thereto an appropriate substance such as serum, plasma, plasma protein (for example, albumin, γ-globulin or the like), or another biological protein. Such a technique is a conventional method, and those skilled in the art can appropriately prepare a calibration curve by preparing HbA1c standard samples suitable for measurement using a whole blood sample.

### EXAMPLES

The present invention is described below concretely by way of Examples. However, the present invention is not limited to the following Examples.

### 1. Confirmation of Hemolysis Caused by Buffer

In a measurement system for a non-pretreated whole blood sample, it is necessary to add whole blood to a latex reagent (hypoosmotic buffer containing latex particles) to complete hemolysis of erythrocytes and adsorption of hemoglobin to the surface of the latex. Hence, we first investigated whether the buffer itself in which the latex is to be suspended has an effect to cause hemolysis of erythrocytes or not.

After placing 2 mL of each solution shown in Table 1 in a tube, 10 µL of whole blood was added to the tube, and hemolysis was visually checked. In the study, 0.15 M NaCl was used as a solution that does not cause hemolysis.

**[Table 1]**

| | Solution used | Result |
|---|---|---|
| 1 | 0.15M NaCl | - |
| 2 | Sample dilution buffer contained in a commercially available kit | + |
| 3 | R1 buffer (buffer to be used for a latex reagent) | +++ |

| | | |
|---|---|---|
| Composition of 2: Hypoosmotic buffer containing as a major component sodium azide. Composition of 3: Hypoosmotic buffer containing as a major component a Good's buffer having the maximum buffer capacity in the neutral to alkaline region. | | |

Hemolysis was also found when a sample dilution buffer contained in a commercially available kit was used, but clear hemolysis did not occur when whole blood immediately after collection was added. However, when whole blood 3 hours after collection was added, no evident difference was found between the sample dilution buffer and R1 buffer. Use of R1 buffer resulted in clear hemolysis even when whole blood immediately after collection was added.

### 2. Measurement of Absorbance of Whole Blood Sample

The following samples were subjected to measurement of the absorbance using a spectrophotometer Ubest-V530 (JASCO).
No. 1: Sample prepared by adding 2 mL of R1 buffer to an HbA1c standard product (freeze-dried product, commercially available) to renature it.
No. 2: Sample prepared by adding 10 µL of whole blood to 2 mL of R1 buffer to cause hemolysis.
No. 3: Sample prepared by dissolving 10 µL of plasma in 2 mL of R1 buffer.

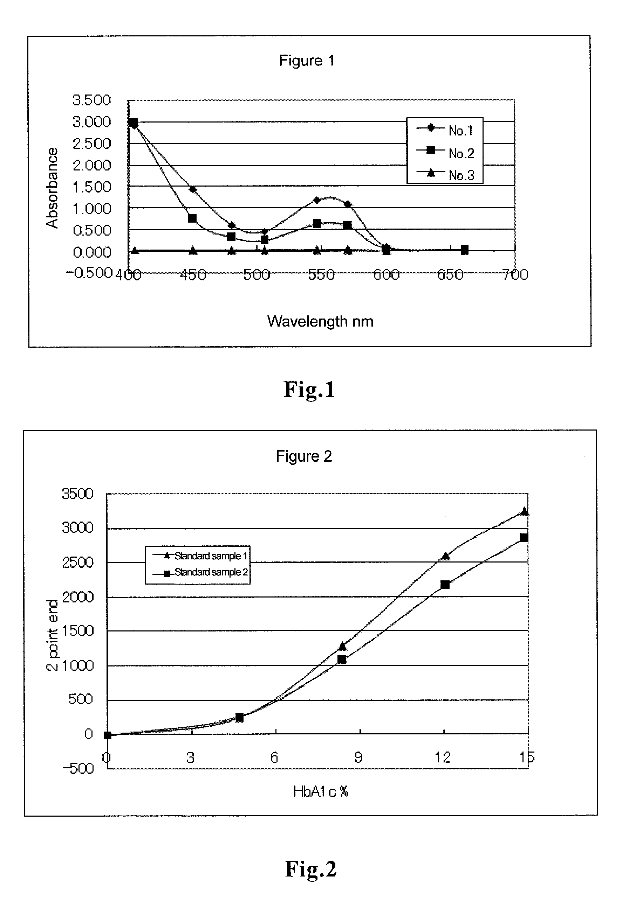

The results are shown in Fig. 1. The concentration of hemoglobin in the hemolysate of the whole blood sample was found to be about half of that in the standard product. The standard product (sample No. 1) simulated a sample prepared by collecting blood cells from the blood cell layer and hemolyzing the blood cells. Since the whole blood sample (sample No. 2) contained plasma, its hemoglobin concentration was lower than that in the sample No. 1. The wavelengths showing the absorbance of hemoglobin pigment were mostly limited to a wavelength range shorter than 600 nm, which indicates that in order to avoid the influence of the absorbance of hemoglobin pigment the wavelength for measurement of the level of latex agglutination needs to be longer than this wavelength range.

### 3. Detection of HbA1c from Non-pretreated Whole Blood Sample by Immunoagglutination Method

Using an HbA1c standard product (freeze-dried product, commercially available), the following two kinds of standard samples (the HbA1c concentration was 0.0% to 14.9%) were prepared, and measurement by the latex agglutination method was carried out using a Hitachi 7170 autoanalyzer to prepare calibration curves (Fig. 2). As an HbA1c-specific antibody, a known anti-HbA1c monoclonal antibody (see Patent Document 1) which was also used in a commercially available kit was used.

### Standard Sample 1:

An HbA1c standard product was renatured with 1 mL of a sample diluent, and 4.8 µL of the resulting dilution was used as a sample.

### Standard Sample 2:

An HbA1c standard product was renatured with 100 µL of a sample diluent, and 12 µL of the resulting dilution was used as a sample.

Using R1 buffer in which latex particles were suspended, 2 µL of a whole blood sample was subjected to measurement by a Hitachi 7170 autoanalyzer. The measured signal was applied to the two kinds of calibration curves shown in Fig. 2 to calculate the HbA1c concentration in the whole blood sample. As a result, the concentration was calculated as shown in Table 2.

**[Table 2]**

| Calibration curve used | HbA1c concentration |
|---|---|
| Standard sample 1 | 4.63% |
| Standard sample 2 | 4.70% |

Blood cells were collected from the blood cell layer of the whole blood sample used herein, hemolyzed, and subjected to measurement by a conventional method. As a result of the calculation, the HbA1c concentration was 5.2%. Although the measured value varied depending on the type of the standard sample used for preparation of the calibration curve, it could be confirmed that a measured value can be obtained even when an immunoassay is directly carried out using a whole blood sample that has not been subjected to pretreatment such as hemolysis or the like. Appropriate preparation of the standard sample enables more accurate measurement.

### 4. Changes in Absorbance during Reaction Process in Detection of HbA1c from Non-pretreated Whole Blood by Immunoagglutination Method

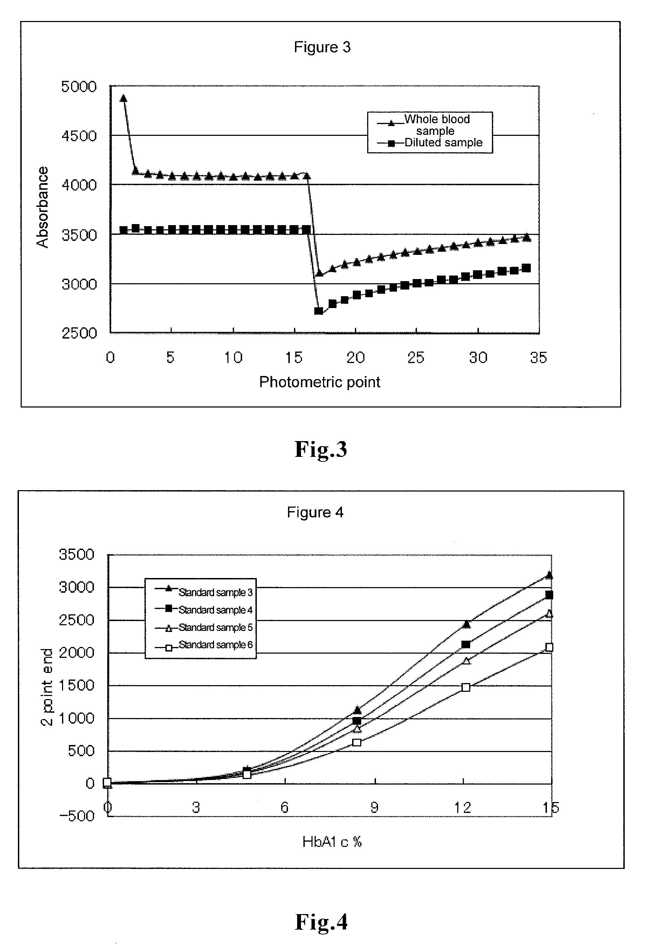

Fig. 3 shows changes in the absorbance during the reaction process in an immunoassay of a diluted sample prepared by hemolyzing blood cells collected from the blood cell layer of a whole blood sample and in a direct immunoassay of a non-pretreated whole blood sample.

The high absorbance at the photometric point 1 in the whole blood sample indicates that hemolysis was in progress. Since the absorbance became almost constant at the photometric point 2, it was shown that the hemolysis proceeded rapidly. During the reaction process, compared to the absorbance of the diluted sample, the absorbance of the whole blood sample was shifted to a higher side to show constantly higher absorbance. The constantly higher absorbance of the whole blood sample was thought to be due to debris after hemolysis of erythrocytes, such as cell membrane. This constantly higher absorbance does not substantially influence the result of the measurement, since the measured value is determined by calculating the difference between absorbances at appropriate points in a section from a point later than the point 16, at which R2 reagent (antibody solution) is added, to the final point 34.

### 5. Preparation of Calibration Curves with HbA1c Standard Samples Prepared by Adding γ-Globulin to Standard Product at Different Concentrations

Using an HbA1c standard product (freeze-dried product, commercially available), the following 4 kinds of standard samples (the HbA1c concentration was 0.0% to 14.9%) were prepared, and 2 µL of each sample was subjected to measurement by the latex agglutination method using a Hitachi 7170 autoanalyzer to prepare calibration curves. The resulting calibration curves are shown in Fig. 4. Standard Sample 3:
An HbA1c standard product was renatured with 100 µL of R1 buffer.

### Standard Sample 4:

An HbA1c standard product was renatured with 100 µL of R1 buffer containing 1.25 mg/mL γ-globulin.

### Standard Sample 5:

An HbA1c standard product was renatured with 100 µL of R1 buffer containing 2.5 mg/mL γ-globulin.

### Standard Sample 6:

An HbA1c standard product was renatured with 100 µL of R1 buffer containing 5 mg/mL γ-globulin.

As the concentration of γ-globulin increased, the signal in the calibration curve decreased. Since the minimum amount of the sample to be injected varies depending on the autoanalyzer, the concentrations of γ-globulin and the like to be added and the amount of the standard sample may be studied and appropriately selected based on the amount (µL) of the non-pretreated whole blood sample and the concentration and the amount of the latex reagent.

## Claims

1. A method of immunoassay of hemoglobin A1c in a sample, said method comprising bringing latex particles into contact with a non-pretreated whole blood sample in a hypotonic solution, and then bringing hemoglobin A1c adsorbed on the latex particles into contact with an anti-hemoglobin A1c antibody.

2. The method according to claim 1, wherein said immunoassay is carried out by an agglutination method.

3. The method according to claim 1 or 2, wherein said hypotonic solution is a buffer containing a Good's buffer at a concentration of 0.02 to 0.2 mol/L, said Good's buffer having the maximum buffer capacity in the neutral to alkaline region.


SEQUENCE LISTING

<110>  TFB, INC.

<120>  Method for immunoassay of hemoglobin A1c in sample

<130>  PF503-PCT

<160>  1

<170>  PatentIn version 3.5

<210>  1
<211>  5
<212>  PRT
<213>  Homo sapiens

<400>  1

Val His Leu Thr Pro
1               5